(19) Europäisches Patentamt
European Patent Office
Office européen des brevets

(11) Publication number: 0 380 956 A2

(12) EUROPEAN PATENT APPLICATION

(21) Application number: 90100833.4

(51) Int. Cl.[5]: A61K 31/557

(22) Date of filing: 16.01.90

(30) Priority: 17.01.89 US 297006

(43) Date of publication of application:
08.08.90 Bulletin 90/32

(84) Designated Contracting States:
AT BE CH DE DK ES FR GB GR IT LI LU NL SE

(71) Applicant: E.R. SQUIBB & SONS, INC.
P.O.Box 4000
Princeton New Jersey 08543-4000(US)

(72) Inventor: Aberg, Gunnar A.K.
519 Bergen Street
Lawrenceville , NJ(US)
Inventor: Ogletree, Martin L.
646 South State Street
Newtown, PA(US)

(74) Representative: Vossius & Partner
Siebertstrasse 4 P.O. Box 86 07 67
D-8000 München 86(DE)

# (54) Method of preventing or treating atherosclerosis using a thromboxane A2 receptor antagonist.

(57) A method is provided for inhibiting or delaying onset of or treating atherosclerosis by administering a thromboxane $A_2$ receptor antagonist which is a 7-oxabicycloheptane prostaglandin analog.

EP 0 380 956 A2

# METHOD OF PREVENTING OR TREATING ATHEROSCLEROSIS USING A THROMBOXANE $A_2$ RECEPTOR ANTAGONIST

The present invention relates to a method for inhibiting onset of or treating atherosclerosis by using a thromboxane $A_2$ receptor antagonist which is a 7-oxabicyclo prostaglandin analog for the preparation of a pharmaceutical drug.

European patent publication 234708 published September 2, 1987 discloses 9-benzyl-1-substituted-alkyl-1,2,3,4-tetrahydrocarbazole derivatives which are useful as thromboxane antagonists, for example, in treating atherosclerosis.

European patent publication 223593 published May 27, 1987 discloses (3-(((sulfonyl)amino)alkyl)-phenoxy)-alkanoic acid derivatives having thromboxane $A^2$ antagonist activity useful for treating atherosclerosis.

Belgian patent application 897763 published March 15, 1984 discloses aromatic methano octenoic acid derivatives which are thromboxane $A_2$ antagonists and may be used in treating atherosclerosis.

European patent publication 253257 published January 20, 1988 discloses acylamino-tetrahydro-naphthyloxy-acetic acid derivatives which are thromboxane $A_2$ antagonists and are useful in treating arteriosclerotic disease.

WO 8702983 published May 21, 1987 discloses N-o-hydroxyphenyl-dioxan-cis-yl-hexenoylsulfonamide derivatives which are antagonists of thromboxane $A_2$ and are useful in treating atherosclerosis.

DE 3518271 published November 27, 1986 discloses 2-(3-hydroxy-1-alkenyl)cyclopropane derivatives which are thromboxane antagonists and are useful for treating atherosclerosis.

European patent publication 201,349 and 201,350 published November 12, 1986 discloses hydroxyphenyl mono-and-di-oxathianyl alkenoic acid derivatives useful as thromboxane $A_2$ antagonists for use in treating atherosclerosis.

European patent publication 150015 published July 31, 1985 discloses 3-N-carbamoyl-2-carboxyalkyl norbornane derivatives useful as thromboxane antagonists for treating atherosclerosis.

Belgian patent application 897763 published March 15, 1984 discloses biphenyl-methoxy-hydroxy-piperidino cyclopentyl heptenoic acid salts useful as thromboxane $A_2$ antagonists and in treating atherosclerosis.

In accordance with the present invention, a method is provided for inhibiting onset of or treating atherosclerosis wherein a therapeutically effective amount of a thromboxane $A_2$ receptor antagonist is systemically administered, such as orally or parenterally, over a prolonged period, to prevent or inhibit onset of or delay or inhibit the development of atherosclerosis during such period of treatment.

The term "thromboxane $A_2$ receptor antagonist" as employed herein includes compounds which are so-called thromboxane $A_2$ receptor antagonists, thromboxane $A_2$ antagonists, thromboxane $A_2$/prostaglandin endoperoxide antagonists, TP-receptor antagonists, or thromboxane antagonists except insofar as the compound is solely an inhibitor of thromboxane synthesis.

The thromboxane $A_2$ receptor antagonist employed herein will be a 7-oxabicycloheptane prostaglandin analog and will include 7-oxabicycloheptane substituted diamide prostaglandin analogs as disclosed in U.S. Patent No. 4,663,336, 7-oxabicycloheptane substituted amino prostaglandin analogs as disclosed in U.S. Patent No. 4,416,896 and 7-oxabicycloheptane prostaglandin analogs as disclosed in U.S. Patent No. 4,537,981. Other 7-oxabicycloheptane prostaglandin analogs may be included as will be apparent to one skilled in the art.

The 7-oxabicycloheptane substituted diamide prostaglandin analogs suitable for use herein, as disclosed in U.S. Patent No. 4,663,336, have the formula

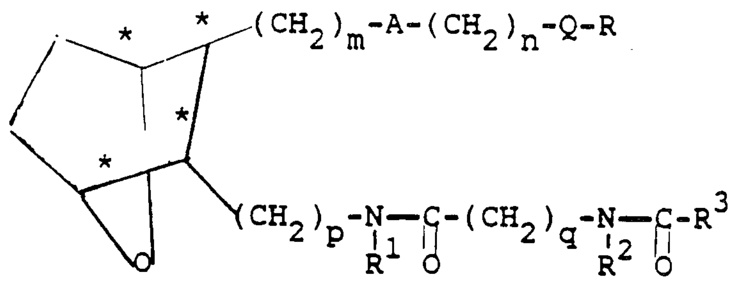

including all stereoisomers thereof, wherein m is 0 to 4; A is -CH=CH- or $-CH_2-CH_2-$; n is 1 to 5; Q is -CH=CH-, $CH_2$ ,

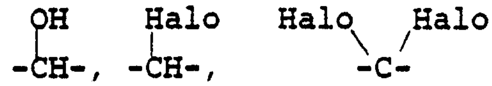

or a single bond; R is $CO_2H$, $CO_2$alkyl, $CO_2$ alkali metal, $CO_2$polyhydroxyamine salt, $-CH_2OH$,

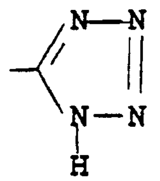

or

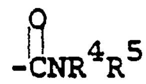

wherein $R^4$ and $R^5$ are the same or different and are H, lower alkyl, hydroxy, lower alkoxy or aryl at least one of $R^4$ and $R^5$ being other than hydroxy and lower alkoxy; p is 1 to 4; $R^1$ is H or lower alkyl; q is 1 to 12; $R^2$ is H or lower alkyl; and $R^3$ is H, lower alkyl, lower alkenyl, lower alkynyl, aryl, arylalkyl, lower alkoxy, arylalkyloxy, aryloxy, amino, alkylamino, arylalkylamino, arylamino,

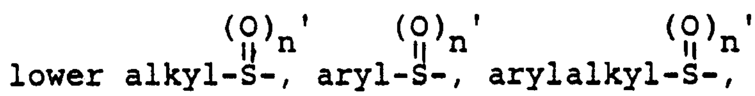

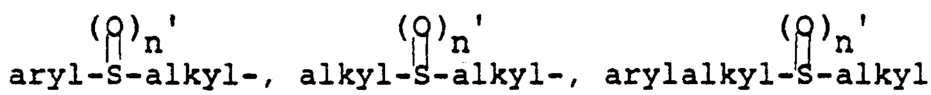

(wherein n′ is 0, 1 or 2), alkylaminoalkyl, arylaminoalkyl, arylalkylaminoalkyl, alkoxyalkyl, aryloxyalkyl or arylalkoxyalkyl.

The 7-oxabicycloheptane substituted amino prostaglandin analogs suitable for use herein, as disclosed in U.S. Patent No. 4,416,896, have the formula

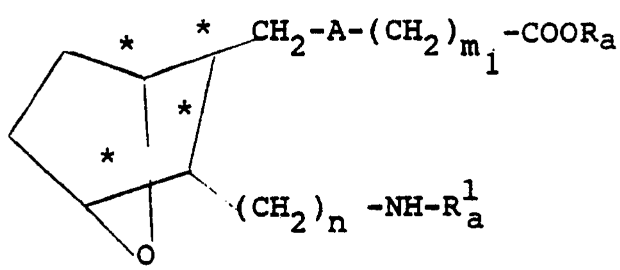

and including all stereoisomers thereof, wherein
A is CH=CH or $(CH_2)_2$; $m_1$ is 1 to 8; $n_1$ is 0 to 5, $R_a$ is H or lower alkyl; and $R_a^1$ is lower alkyl, aryl, aralkyl, lower alkoxy, aralkoxy or

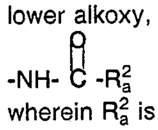

wherein $R_a^2$ is lower alkyl, aryl, aralkyl, alkoxy, aryloxy, aralkoxy, alkylamino, arylamino or aralkylamino.

The 7-oxabicycloheptane prostaglandin analogs suitable for use herein, as disclosed in U.S. Patent No. 4,537,981, have the formula

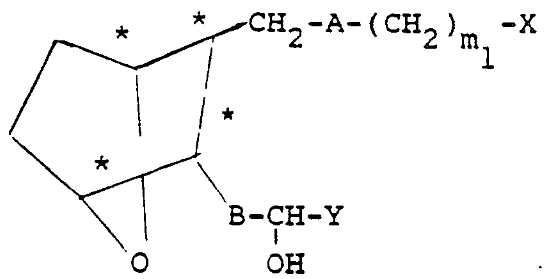

and including all stereoisomers thereof, wherein A and B may be the same or different and A is CH = CH or $(CH_2)_2$; B is CH = CH, C≡C or $(CH_2)_2$; $m_1$ is 1 to 8;
X is OH;

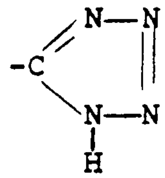 ;

$CO_2R_a$ wherein $R_a$ is H or lower alkyl; or

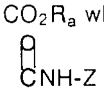

wherein Z is H, lower alkyl, aryl, $SO_2$-$Q_1$ (with $Q_1$ being lower alkyl or aryl),

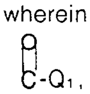

or $OR_b^2$ wherein $R_b^2$ is H, and Y is alkyl, substituted alkyl; aryl-lower alkyl; alkenyl; alkynyl, aryl; pyridyl; substituted pyridyl; pyridyl-lower alkyl; thienyl, substituted thienyl; thienyl-lower alkyl; cycloalkyl; cycloalkylalkyl; substituted cycloalkylalkyl; or phenoxymethyl.

Preferred examples of thromboxane $A_2$ receptor antagonists which may be employed herein include the 7-oxabicycloheptane compounds disclosed in U.S. Patent No. 4,537,981, especially, [1S- [1α,2β(5Z),3β-(1E,3R,4S),4α]]-7-[3-(3-hydroxy-4-phenyl-1-pentenyl)-7-oxabicyclo-[2.2.1]hept-2-yl]-5-heptenoic acid; the 7-oxabicycloheptane substituted amino-prostaglandin analogs disclosed in U.S. Patent No. 4,416,896, especially, [1S-[1α,2β,(5Z),3β,4α]]-7-[3-[[2-(phenylamino)carbonyl]hydrazino]methyl]-7-oxabicyclo[2.2.1]hept-2-yl]-5-heptenoic acid; the 7-oxabicycloheptane substituted diamide prostaglandin analogs disclosed in U.S. Patent No. 4,663,336, expecially, [1S-[1β,2α(5Z),3α,4β]]-7-[3-[[[[(1-oxoheptyl)amino]acetyl]amino]methyl]-7-oxabicyclo[2.2.1]hept-2-yl]-5-heptenoic acid and the corresponding tetrazole, and [1S-[1α,2β(Z),3β,4α]]-7-[3-[[[[(4-cyclohexyl-1-oxobutyl)amino]acetyl]amino]methyl]-7-oxabicyclo[2.2.1]hept-2-yl]-5-heptenoic acid.

The disclosures of the above-mentioned patents are incorporated herein by reference.

In carrying out the method of the present invention, the thromboxane $A_2$ receptor antagonist may be administered systemically, such as orally or parenterally, to mammalian species, such as monkeys, dogs, cats, rats, humans, etc.

The thromboxane $A_2$ receptor antagonist may be incorporated in a conventional dosage form, such as a tablet, capsule, elixir or injectable. The above dosage forms will also include the necessary carrier material, excipient, lubricant, buffer, antibacterial, bulking agent (such as mannitol), anti-oxidants (ascorbic acid or sodium bisulfite) or the like. Oral dosage forms are preferred, although parenteral forms are quite satisfactory as well.

With regard to such systemic formulations, single or divided doses of from about 0.5 to about 2500 mg, preferably from about 5 to 200 mg/one to four times daily, may be administered in systemic dosage forms as described above for a prolonged period, that is, for as long as the potential for onset of atherosclerosis remains or the symptoms of atherosclerosis continue. Sustained release forms of such formulations which may provide such amounts biweekly, weekly, monthly and the like may also be employed. A dosing period of at least one week is required to achieve minimal benefit.

The following Examples represent preferred embodiments of the present invention.

Example 1

An injectable solution of thromboxane $A_2$ receptor antagonist for intravenous use in treating or delaying onset of atherosclerosis is produced as follows:

| | |
|---|---|
| [1S-[1$\alpha$,2$\beta$(5Z),3$\beta$,4$\alpha$]]-7-[3-[[2-(phenylamino)carbonyl]hydrazino]-methyl]-7-oxabicyclo[2.2.1]hept-2-yl]-5-heptenoic acid (SQ 29,548) | 2500 mg |
| Methyl paraben | 5 mg |
| Propyl paraben | 1 mg |
| Sodium chloride | 25 g |
| Water for injection qs. | 5 l. |

The thromboxane $A_2$ receptor antagonist, preservatives and sodium chloride are dissolved in 3 liters of water for injection and then the volume is brought up to 5 liters. The solution is filtered through a sterile filter and aseptically filled into presterilized vials which are then closed with presterilized rubber closures. Each vial contains a concentration of 75 mg of active ingredient per 150 ml of solution.

## Example 2

An injectable for use in delaying onset of or treating atherosclerosis is prepared as described in Example 1 except that the thromboxane $A_2$ receptor antagonist employed is [1S-[1α,2β(5Z),3β(1E,3R,4S)-,4α]]-7-[3-(3-hydroxy-4-phenyl-1-pentenyl)-7-oxabicyclo[2.2.1]hept-2-yl]-5-heptenoic acid (SQ 28,668).

## Example 3

An injectable solution of thromboxane $A_2$ receptor antagonist for use in delaying onset of or treating atherosclerosis containing [1S-[1β,2α(5Z),3α,4β]]-7-[3-[[[[(1-oxoheptyl)amino]acetyl]amino]methyl]-7-oxabicyclo[2.2.1]hept-2-yl]-5-heptenoic acid (SQ 30,741) as the thromboxane $A_2$ receptor antagonist is prepared as described in Example 1.

## Example 4

An injectable for use in delaying onset of or treating atherosclerosis is prepared as described in Example 1 except that the thromboxane $A_2$ receptor antagonist employed is [1S-[lα,2β(Z),3β,4α]]-7-[3-[[[[(4-cyclohexyl-1-oxobutyl)amino]acetyl]amino]methyl]-7-oxabicyclo[2.2.1]hept-2-yl]-5-heptenoic acid.

## Example 5

A thromboxane $A_2$ antagonist formulation suitable for oral administration for use in delaying onset of or treating atherosclerosis is set out below.

1000 tablets each containing 400 mg of thromboxane $A_2$ receptor antagonist are produced from the following ingredients.

| | |
|---|---|
| [1S-[1$\beta$,2$\alpha$(5Z),3$\alpha$,4$\beta$]]-7-[3-[[[[(1-Oxoheptyl)amino]acetyl]amino]methyl]-7-oxabicyclo[2.2.1]hept-2-yl]-5-heptenoic acid (SQ 30,741) | 400 g |
| Corn Starch | 50 g |
| Gelatin | 7.5 g |
| Avicel (microcrystalline cellulose) | 25 g |
| Magnesium stearate | 2.5 g |

The thromboxane $A_2$ receptor antagonist and corn starch are admixed with an aqueous solution of the gelatin. The mixture is dried and ground to a fine powder. The Avicel and then the magnesium stearate are admixed with the granulation. This is then compressed in a tablet to form 1000 tablets each containing 400 mg of active ingredient.

Example 6

A thromboxane $A_2$ antagonist tablet formulation for use in delaying onset of or treating atherosclerosis is prepared as described in Example 5 except that SQ 29,548 is employed as the thromboxane $A_2$ receptor antagonist in place of SQ 30,741.

Example 7

A thromboxane $A_2$ antagonist tablet formulation for use in delaying onset of or treating atherosclerosis is prepared as described in Example 5 except that SQ 28,668 is employed in place of SQ 30,741.

**Claims**

1. Use of a thromboxane $A_2$ receptor antagonist, which thromboxane $A_2$ receptor antagonist is a 7-oxabicycloheptane prostaglandin analog for the preparation of pharmaceutical compositions.

2. Use as defined in Claim 1 wherein the 7-oxabicycloheptane prostaglandin analog is a 7-oxabicycloheptane substituted diamide prostaglandin analog or a 7-oxabicycloheptane substituted amino prostaglandin analog.

3. Use as defined in Claim 1 wherein the 7-oxabicycloheptane prostaglandin analog has the formula

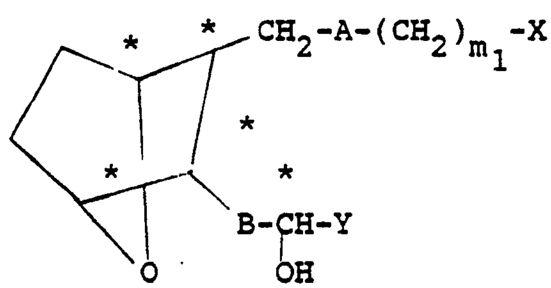

and including all stereoisomers thereof, wherein
A and B may be the same or different and A is
CH = CH or $(CH_2)_2$; B is CH = CH, C≡C or
$(CH_2)_2$; $m_1$ is 1 to 8; X is OH;

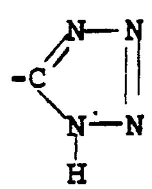 ;

$CO_2R_a$ wherein $R_a$ is H or lower alkyl; or

$\overset{O}{\overset{\|}{C}}NH\text{-}Z$

wherein Z is H, lower alkyl, aryl, $SO_2$-$Q_1$ (with $Q_1$ being lower alkyl or aryl),

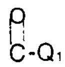

or $OR_b^2$ wherein $R_b^2$ is H, and Y is alkyl; substituted alkyl; aryl-lower alkyl; alkenyl; alkynyl, aryl; pyridyl; substituted pyridyl; pyridyl-lower alkyl; thienyl, substituted thienyl; thienyl-lower alkyl; cycloalkyl; cycloalkylalkyl; substituted cycloalkylalkyl; or phenoxymethyl.

4. Use as defined in Claim 2 wherein the 7-oxabicycloheptane substituted diamide prostaglandin analog has the formula

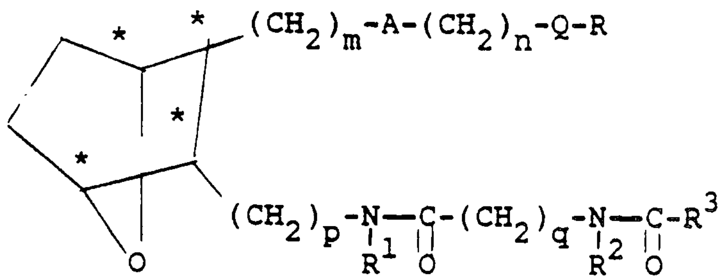

including all stereoisomers thereof, wherein m is 0 to 4; A is -CH=CH- or $-CH_2-CH_2-$; n is 1 to 5; Q is -CH=CH-, $-CH_2$,

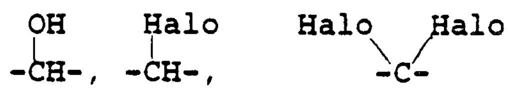 ,

or a single bond; R is $CO_2H$, $CO_2$alkyl, $CO_2$ alkali metal, $CO_2$polyhydroxyamine salt, $-CH_2OH$,

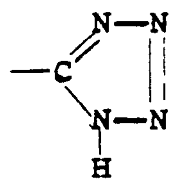 or 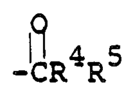

wherein $R^4$ and $R^5$ are the same or different and are H, lower alkyl, hydroxy, lower alkoxy or aryl at least one of $R^4$ and $R^5$ being other than hydroxy and lower alkoxy; p is 1 to 4; $R^1$ is H or lower alkyl; q is 1 to 12; $R^2$ is H or lower alkyl; and $R^3$ is H, lower alkyl, lower alkenyl, lower alkynyl, aryl, arylalkyl, lower alkoxy, arylalkyloxy, aryloxy, amino, alkylamino, arylalkylamino, arylamino,

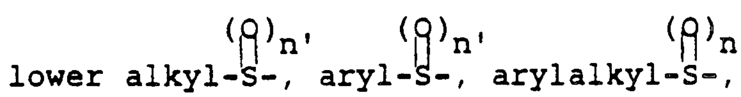

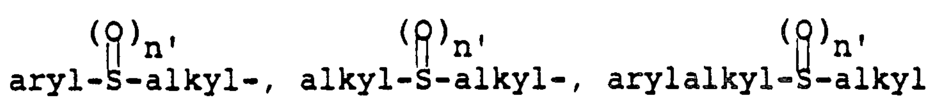

(wherein $n'$ is 0, 1 or 2), alkylaminoalkyl, arylaminoalkyl, arylalkylaminoalkyl, alkoxyalkyl, aryloxyalkyl or arylalkoxyalkyl.

5. Use as defined in Claim 2 wherein the 7-oxabicycloheptane substituted amino prostaglandin analog has the formula

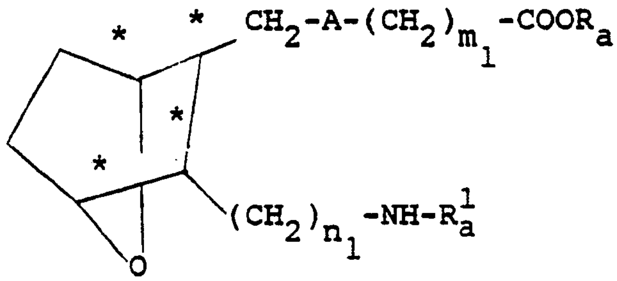

and including all stereoisomers thereof, wherein A is CH=CH or $(CH_2)_2$; $m_1$ is 1 to 8; $n_1$ is 0 to 5, $R_a$ is H or lower alkyl; and $R_a^1$ is lower alkyl, aryl, aralkyl, lower alkoxy, aralkoxy or

$$-NH-\overset{O}{\overset{\|}{C}}-R_a^2$$

wherein $R_a^2$ is lower alkyl, aryl, aralkyl, alkoxy, aryloxy, aralkoxy, alkylamino, arylamino or aralkylamino.

6. Use as defined in Claim 1 wherein the thromboxane $A_2$ receptor antagonist is [1S-[1α,2β(5Z),3β-(1E,3R,4S),4α]]-7-[3-(3-hydroxy-4-phenyl-1-pentenyl)-7-oxabicyclo[2.2.1]hept-2-yl]-5-heptenoic acid.

7. Use as defined in Claim 1 wherein the thromboxane $A_2$ receptor antagonist has the name [1S-[1β,2α-(5Z),3α,4β]]-7-[3-[[[[(1-oxoheptyl)amino]acetyl]amino]methyl]-7-oxabicyclo[2.2.1]hept-2-yl]-5-heptenoic acid or the corresponding tetrazole.

8. Use as defined in Claim 1 wherein the thromboxane $A_2$ receptor antagonist has the name [1S-[1α,2β-(Z),3β,4α]]-7-[3-[[[[(4-cyclohexyl-1-oxobutyl)amino]acetyl]amino]methyl]-7-oxabicyclo[2.2.1]hept-2-yl]-5-heptenoic acid.

9. Use as defined in Claim 1 wherein the thromboxane $A_2$ receptor antagonist has the name [1S-[1α,2β-(5Z),3β,4α]]-7-[3-[[2-(phenylamino)carbonyl]hydrazino]methyl]-7-oxabicyclo[2.2.1]hept-2-yl]-5-heptenoic acid.

10. Use as defined in Claim 1 wherein the pharmaceutical composition is in a dosage form for oral or parenteral administration.